(19) Europäisches Patentamt
European Patent Office
Office européen des brevets

(11) EP 3 434 284 A1

(12) **EUROPEAN PATENT APPLICATION**
published in accordance with Art. 153(4) EPC

(43) Date of publication:
**30.01.2019 Bulletin 2019/05**

(21) Application number: **17770248.7**

(22) Date of filing: **22.03.2017**

(51) Int Cl.:
***A61K 45/00*** *(2006.01)* ***A61K 31/40*** *(2006.01)*
***A61K 31/4178*** *(2006.01)* ***A61K 45/06*** *(2006.01)*
***A61P 3/10*** *(2006.01)* ***A61P 5/42*** *(2006.01)*
***A61P 13/12*** *(2006.01)* ***A61P 43/00*** *(2006.01)*

(86) International application number:
**PCT/JP2017/011340**

(87) International publication number:
**WO 2017/164208 (28.09.2017 Gazette 2017/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.03.2016 JP 2016059376**
**31.01.2017 JP 2017015438**

(71) Applicant: **Daiichi Sankyo Company, Limited**
**Tokyo 103-8426 (JP)**

(72) Inventors:
- **ARAI, Kiyoshi**
  **Tokyo 103-8426 (JP)**
- **HOMMA, Tsuyoshi**
  **Tokyo 103-8426 (JP)**
- **SAWANOBORI, Tomoko**
  **Tokyo 103-8426 (JP)**
- **NAKAJIMA, Shin**
  **Tokyo 103-8426 (JP)**
- **HISATOMI, Rie**
  **Tokyo 103-8426 (JP)**
- **YOSHIMURA, Motonobu**
  **Tokyo 103-8426 (JP)**

(74) Representative: **Fairbairn, Angus Chisholm**
**Marks & Clerk LLP**
**90 Long Acre**
**London WC2E 9RA (GB)**

# (54) MEDICINE FOR TREATING RENAL DISEASE

(57) Provided is a pharmaceutical composition for treating a renal disease. The pharmaceutical composition for treating a renal disease comprises a mineralocorticoid receptor antagonist.

EP 3 434 284 A1

## Description

Technical Field

The present invention relates to a pharmaceutical composition comprising a mineralocorticoid receptor (aldosterone receptor: MR) antagonist for treating a renal disease, a method for treating a renal disease with the MR antagonist, and combination use or a combination formulation of the MR receptor antagonist and a drug having an antihypertensive agent for treating a renal disease.

Background Art

As the disease state of a renal disease progresses, the renal function lowers, and the renal disease shifts to chronic renal failure, which requires treatment by hemodialysis. Particularly, the commonest cause of starting hemodialysis therapy is diabetic nephropathy. Hemodialysis patients are increasing year by year, which is currently now a major medical problem. Under such circumstances, the therapeutic aim for renal diseases is not only to avoid and delay dialysis but also to inhibit the onset of cardiovascular diseases to maintain a quality of daily life similar to that of healthy individuals and to ensure healthy life expectancy. Therefore, it is an urgent objective to develop a therapeutic agent for renal diseases.

In recent years, it has also been known that remission of albuminuria to normal levels leads to inhibition of the progression of renal diseases and inhibition of cardiovascular events. Therefore, expectations for remission treatment by a therapeutic agent for renal diseases have been increasing.

The mineralocorticoid receptor is known to play an important role in controlling the electrolyte balance and blood pressure in the body. As renal diseases involving the mineralocorticoid receptor, diabetic nephropathy (Non-Patent Literature 1) and glomerulonephritis (such as IgA nephropathy or mesangioproliferative glomerulonephritis) (Non-Patent Literatures 2 and 3), nephrosclerosis (Non-Patent Literature 4), and the like are known.

The mineralocorticoid receptor is known to play an important role in controlling the electrolyte balance and blood pressure in the body, and MR antagonists having a steroid structure such as spironolactone and eplerenone are known to be useful for the treatment of hypertension and heart failure. Furthermore, 1-(2-hydroxyethyl)-4-methyl-N-[4-(methylsulfonyl)phenyl]-5-[2-(trifluoromethyl)phenyl]-1H-pyrrole-3-carboxamide (hereinafter referred to as compound (I)) which is an MR antagonist, is disclosed in Patent Literatures 1 and 2.

Angiotensin II receptor antagonists and calcium antagonists are widely used as medicines for the treatment or prevention of hypertension and the like.

Angiotensin II receptor antagonists, which are inhibitors of the renin-angiotensin system, are particularly effective for renin-dependent hypertension and show a protective effect against cardiovascular disorders and renal disorders. Calcium antagonists are also effective for hypertension with body fluid retention (renin-independent hypertension), due to their natriuretic effect in addition to their vasodilatation by antagonizing (inhibiting) the function of the calcium channel.

(5-Methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate (hereinafter referred to as olmesartan medoxomil), which is an angiotensin II receptor antagonist, is known to be useful as a medicine for treating or preventing hypertension, cardiac diseases and the like (Patent Literature 3).

Olmesartan medoxomil is commercially available as Olmetec (R) tablets or Benicar (R) which contains 5 mg, 10 mg, 20 mg or 40 mg of olmesartan medoxomil as an active ingredient and contains low substituted hydroxypropylcellulose, hydroxypropylcellulose, crystalline cellulose, lactose and magnesium stearate as additives.

3-Ethyl-5-methyl-(±)-2-[(2-aminoethoxy)methyl]-4-(2-chlorophenyl)-1,4-dihydro-6-methylpyridine-3,5-dicarboxylate (hereinafter referred to as amlodipine) is a known compound which is useful as an excellent calcium antagonist and a medicine for treating or preventing hypertension, cardiac diseases and the like (Patent Literature 4).

Amlodipine is commercially available as Norvasc (R) tablets which contain 3.47 mg or 6.93 mg of amlodipine besylate (2.5 mg or 5 mg as amlodipine) as active ingredient and contains crystalline cellulose, anhydrous calcium hydrogen phosphate, sodium carboxymethyl starch, magnesium stearate, hydroxypropylmethyl cellulose, titanium oxide, talc and carnauba wax as additives.

Citation List

Patent Literature

Patent Literature 1: International Publication WO 2006/012642 (U.S. Publication US 2008-0234270)
Patent Literature 2: International Publication WO 2008/056907 (U.S. Publication US 2010-0093826)

Patent Literature 3: Japanese Patent No. 2082519 (U.S. Pat. No. 5, 616, 599)
Patent Literature 4: Japanese Patent No. 1401088 (U.S. Pat. No. 4,572,909)

Non-Patent Literatures

Non-Patent Literature 1: Nephron. Experimental Nephrology 2014; 126: 16-24
Non-Patent Literature 2: Journal of Nephrology 2013; 26: 199-206
Non-Patent Literature 3: Journal of Translational Medicine 2011; 9:169
Non-Patent Literature 4: American Journal of Nephrology 2004; 24: 242-9

Summary of Invention

Technical Problem

The present inventors have conducted diligent research for the purpose of developing excellent therapeutic agents for renal diseases. As a result, the inventors have found that the purpose is achieved by a medicine containing a mineralocorticoid receptor antagonist as an active ingredient, thereby completing the present invention.

Solution to Problem

The present inventors have conducted diligent research in order to solve the above-mentioned problem, and as a result, have found that a particular MR antagonist has an excellent therapeutic effect on renal diseases involving the mineralocorticoid receptor (particularly, diabetic nephropathy). The present inventors have also found that such a medicine is effective for remission treatment of a renal disease involving the mineralocorticoid receptor (particularly, diabetic nephropathy). The present invention has been completed based on the above findings.
Specifically, the present invention includes the following aspects of the invention:

(1) A pharmaceutical composition comprising a mineralocorticoid receptor antagonist or a pharmaceutically acceptable salt thereof, or a hydrate thereof, for treating a renal disease by ameliorating a renal disorder involving activation of the mineralocorticoid receptor;
(2) A pharmaceutical composition comprising a mineralocorticoid receptor antagonist or a pharmaceutically acceptable salt thereof, or a hydrate thereof, for treating a renal disease to put it into a remission state by ameliorating a renal disorder involving activation of the mineralocorticoid receptor;
(3) The pharmaceutical composition according to the above (1) or (2), wherein the renal disease is diabetic nephropathy, glomerulonephritis or nephrosclerosis;
(4) The pharmaceutical composition according to the above (1) or (2), wherein the renal disease is diabetic nephropathy;
(5) The pharmaceutical composition according to any one of the above (1) to (4), wherein the mineralocorticoid receptor antagonist is (S)-1-(2-hydroxyethyl)-4-methyl-N-[4-(methylsulfonyl)phenyl]-5-[2-(trifluoromethyl)phenyl]-1H-pyrrole-3-carboxamide;
(5-1) The pharmaceutical composition according to any one of the above (1) to (4), wherein the mineralocorticoid receptor antagonist is (S)-1-(2-hydroxyethyl)-4-methyl-N-[4-(methylsulfonyl)phenyl]-5-[2-(trifluoromethyl)phenyl]-1H-pyrrole-3-carboxamide, spironolactone or eplerenone;
(6) The pharmaceutical composition according to any one of the above (1) to (5), further comprising one or more components selected from an angiotensin II receptor antagonist and a calcium antagonist;
(6-1) A pharmaceutical composition comprising a mineralocorticoid receptor antagonist or a pharmaceutically acceptable salt thereof, or a hydrate thereof in combination with a drug containing one or more components selected from an angiotensin II receptor antagonist and a calcium antagonist;
(7) The pharmaceutical composition according to the above (6), wherein the one or more components selected from an angiotensin II receptor antagonist and a calcium antagonist is olmesartan medoxomil;
(8) The pharmaceutical composition according to the above (6), wherein the one or more components selected from an angiotensin II receptor antagonist and a calcium antagonist is amlodipine;
(9) A method for treating a renal disease to put it into a remission state by ameliorating a renal disorder involving activation of the mineralocorticoid receptor, comprising administering a mineralocorticoid receptor antagonist to a mammal; and
(10) The method according to the above (9), comprising administering to the mammal one or more components

selected from an angiotensin II receptor antagonist and a calcium antagonist, concurrently with or separately from the mineralocorticoid receptor antagonist.

Advantageous Effects of Invention

A medicine containing an MR antagonist as an active ingredient according to the present invention is useful as a medicine for remission treatment of a renal disease, because it exhibits an excellent ameliorative effect on the renal disease (preferably diabetic nephropathy). The above medicine is preferably a medicine for mammals, more preferably for humans.

Description of Embodiments

The medicine of the present invention is characterized by containing as an active ingredient an MR receptor antagonist comprising a substance selected from the group consisting of the compound represented by the formula (I) and atropisomers thereof, and pharmaceutically acceptable salts thereof.

The compound represented by the above formula (I) and atropisomers thereof, and pharmaceutically acceptable salts thereof as an active ingredient of the medicine of the present invention are known, and can be produced, for example, according to such methods as described in International Publication WO 2006/012642 (U.S. Publication US 2008-0234270), International Publication WO 2008/056907 (U.S. Publication US 2010-0093826) or the like. Compound (I) or its atropisomer is preferably the following atropisomer compound (Ia) (chemical name: (S)-1-(2-hydroxyethyl)-4-methyl-N-[4-(methylsulfonyl)phenyl]-5-[2-(trifluoromethyl)phenyl]-1H-pyrrole-3-carboxamide).

[Formula 1]

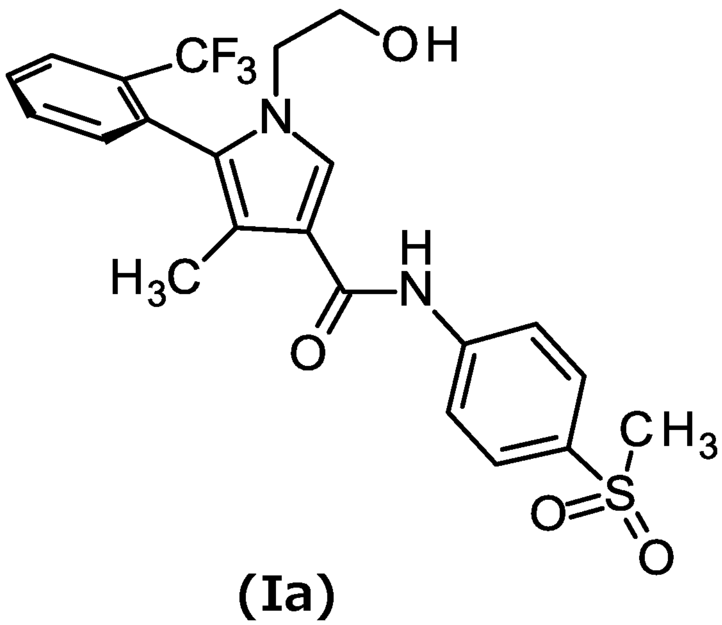

(Ia)

As the substance selected from compound (I) and its atropisomers, a hydrate or a solvate thereof may be also used. Compound (I) can also be used in the form of a pure atropisomer or any mixture of atropisomers.

In the present invention, examples of the "angiotensin II receptor antagonist" include a biphenyltetrazole compound such as olmesartan medoxomil, olmesartan cilexetil, losartan, candesartan cilexetil, valsartan or irbesartan; a biphenylcarboxylic acid compound such as telmisartan; eprosartan or azilsartan; preferably a biphenyltetrazole compound; more preferably olmesartan medoxomil, losartan, candesartan cilexetil, valsartan or irbesartan; particularly preferably olmesartan medoxomil, losartan or candesartan cilexetil; and most preferably olmesartan medoxomil.

Olmesartan medoxomil is described in Japanese Unexamined Patent Application Publication No. H05-78328, U.S. Patent No. 5,616,599 and the like, and its chemical name is (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate. The olmesartan medoxomil of the present application includes its pharmacologically acceptable salts.

Losartan (DUP-753) is described in Japanese Unexamined Patent Application Publication No. S63-23868, U.S. Patent No. 5,138,069 and the like, and its chemical name is 2-butyl-4-chloro-1-[2'-(1H-tetrazol-5-yl)biphenyl-4-ylmethyl]-1H-imidazole-5-methanol. The losartan of the present application includes its pharmacologically acceptable salts (such as losartan potassium salt).

Candesartan cilexetil is described in Japanese Unexamined Patent Application Publication No. H04-364171, an EP-459136, U.S. Patent No. 5,354,766 and the like, and its chemical name is 1-(cyclohexyloxycarbonyloxy)ethyl-2-ethoxy-1-[2'-(1H-tetrazol-5-yl)biphenyl-4-ylmethyl]-1H-benzimidazole-7-carboxylate. The candesartan cilexetil of the present application includes its pharmacologically acceptable salts.

Valsartan (CGP-48933) is described in Japanese Unexamined Patent Application Publication No. H04-159718, EP-433983 and the like, and its chemical name is (S)-N-valeryl-N-[2'-(1H-tetrazol-5-yl)biphenyl-4-ylmethyl)valine. The valsartan of the present application includes its pharmacologically acceptable esters or pharmacologically acceptable salts.

Irbesartan (SR-47436) is described in Japanese Unexamined Patent Application Publication (Translation of PCT Application) No. H04-506222, WO 91-14679 and the like, and its chemical name is 2-N-butyl-4-spirocyclopentane-1-[2'-(tetrazol-5-yl)biphenyl-4-ylmethyl]-2-imidazolin-5-one. The irbesartan of the present application includes its pharmacologically acceptable salts.

Eprosartan (SKB-108566) is described in U.S. Patent No. 5,185,351 and the like, and its chemical name is 3-[1-(4-carboxyphenylmethyl)-2-n-butyl-imidazol-5-yl]-2-thienyl-methyl-2-propenoic acid. The eprosartan of the present application includes its carboxylic acid derivatives, pharmacologically acceptable esters of the carboxylic acid derivatives or pharmacologically acceptable salts thereof (such as eprosartan mesylate).

Telmisartan (BIBR-277) is described in U.S. Patent No. 5,591,762 and the like, and its chemical name is 4'-[[4-methyl-6-(1-methyl-2-benzimidazolyl)-2-propyl-1-benzimidazolyl]methyl]-2-biphenylcarboxylic acid. The telmisartan of the present application includes its carboxylic acid derivatives, pharmacologically acceptable esters of the carboxylic acid derivatives or pharmacologically acceptable salts thereof.

Azilsartan is described in Japanese Unexamined Patent Application Publication No. H05-271228, U.S. Patent No. 5,243,054 and the like, and its chemical name is 2-ethoxy-1{[2'-(5-oxo-4,5-dihydro-1,2,4-oxadiazol-3-yl)biphenyl-4-yl]methyl}-1H-benzo[d]imidazole-7-carboxylic acid.

When each of the above compounds has an asymmetric carbon, each of angiotensin II receptor antagonists of the present invention also includes optical isomers and a mixture of isomers thereof. Furthermore, hydrates of the above compounds are included.

In the present invention, "calcium antagonist" is a calcium antagonist comprising a substance selected from the group consisting of 1,4-dihydropyridine-based compounds and pharmacologically acceptable salts thereof.

As calcium antagonists comprising a 1,4-dihydropyridine-based compound, various drugs have been proposed and actually used in clinical practice, and those skilled in the art can thus select an appropriate drug that exhibits the effect of the present invention. Examples of calcium antagonists comprising a 1,4-dihydropyridine-based compound that can be used include, but are not limited to, azelnidipine, amlodipine, benidipine, nitrendipine, manidipine, nicardipine, nifedipine, nisoldipine, cilnidipine, lercanidipine, niguldipine, nimodipine, aranidipine, efonidipine, barnidipine, felodipine, clevidipine, lacidipine and nilvadipine.

The type of pharmacologically acceptable salt of the 1,4-dihydropyridine-based compound is not particularly limited, and can be appropriately selected by those skilled in the art. The pharmacologically acceptable salt may be either an acid addition salt or a base addition salt. Examples of the pharmacologically acceptable salts include, but are not limited to, an alkali metal salt such as a sodium salt, a potassium salt or a lithium salt, an alkaline earth metal salt such as a calcium salt or a magnesium salt and a metal salt such as an aluminum salt, an iron salt, a zinc salt, a copper salt, a nickel salt or a cobalt salt; a base addition salt such as an inorganic salt such as an ammonium salt or an amine salt such as an organic salt such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl phenethylamine salt, a piperazine salt, a tetramethylammonium salt, tris(hydroxymethyl)aminomethane salt; or alternatively a mineral acid salt such as a hydrofluoride, a hydrochloride, a hydrobromide, a hydroiodide, a nitrate, a perchlorate, a sulfate or a phosphate; a sulfonate such as a methanesulfonate, a trifluoromethanesulfonate, a ethanesulfonate, a benzenesulfonate or a p-toluenesulfonate; a carboxylate such as a besylate, a fumarate, a succinate, a citrate, a tartrate, an oxalate or a maleate; and an amino acid salt such as a glutamate or an aspartate.

As the calcium antagonist comprising a 1,4-dihydropyridine-based compound, hydrates or solvates of the above compounds or pharmacologically acceptable salts thereof may be used. The calcium antagonist comprising a 1,4-dihydropyridine-based compound may have also one or more asymmetric carbons in the molecule, and optical isomers in a pure form based on the asymmetric carbon(s) or stereoisomers such as diastereoisomers, or any mixture of stereoisomers or a racemate or the like can be used, but (±)-2-amino-1,4-dihydro-6-methyl-4-(3-nitrophenyl)-3,5-pyridinedicarboxylic acid 3-(1-diphenylmethylazetidin-3-yl) ester 5-isopropyl ester is preferred.

The calcium antagonist comprising a 1,4-dihydropyridine-based compound is more preferably amlodipine,

which can easily be produced according to the method described in Japanese Patent No. 1401088 (US Pat. No. 4,572,909) and the like. Amlodipine can form pharmacologically acceptable salts, and these salts are also included in the present invention. The pharmacologically acceptable salt may be either an acid addition salt or a base addition salt. Examples of the pharmacologically acceptable salts include, but are not limited to, a besylate, a hydrochloride, a hydrobromide, a fumarate, a citrate, a tartrate, a maleate, a camsylate, a lactate, a mesylate, a nicotinate and a gluconate, and preferably a besylate.

In the present invention, the phrase "comprising a mineralocorticoid receptor antagonist or a pharmaceutically acceptable salt thereof, or a hydrate thereof in combination with a drug containing one or more components selected from an angiotensin II receptor antagonist and a calcium antagonist" refers to an aspect in which a formulation containing a mineralocorticoid receptor antagonist or a pharmaceutically acceptable salt thereof, or a hydrate thereof is administered concurrently with or separately from a formulation containing one or more components selected from an angiotensin II receptor antagonist and a calcium antagonist, or alternatively an aspect in which a formulation containing a mineralocorticoid receptor antagonist or a pharmaceutically acceptable salt thereof and one or more components selected from an angiotensin II receptor antagonist and a calcium antagonist (hereinafter referred to as "combination formulation") is administered.

In the present invention, administering "concurrently with" is not particularly limited as long as the formulations are in dosage forms that can be administered at approximately the same time, but they are preferably administered as a single composition.

In the present invention, administering "separately from" refers to administering separately at different times. For example, a component selected from an angiotensin II receptor antagonist and a calcium antagonist is first administered, and a mineralocorticoid receptor antagonist or a pharmaceutically acceptable salt thereof, or a hydrate thereof is then administered after a predetermined period of time, or vice versa.

An MR antagonist which is an active ingredient of the pharmaceutical composition of the present invention and an angiotensin II receptor antagonist, a calcium antagonist or a diuretic agent each are independently administered in separate unit dosage forms, or they can be blended to prepare a physically single unit dosage form.

When using the pharmaceutical composition of the present invention as a prophylactic or therapeutic agent for the above diseases, an MR antagonist which is an active ingredient of the pharmaceutical composition of the present invention, and an angiotensin II receptor antagonist, a calcium antagonist or a diuretic agent each can be orally or parenterally administered in themselves or in a dosage form such as a tablet, a capsule, granules, a powder or a syrup in the case of oral administration, or an injection or a suppository in the case of parenteral administration, which is produced according to a well-known method using appropriate pharmaceutically acceptable additives such as excipients, lubricants, binders, disintegrants, emulsifiers, stabilizers, flavoring agents or diluents. However, since the active ingredient contained in the medicine of the present invention is a drug which is generally administered orally, the medicine of the present invention is desirably administered orally.

The pharmaceutical composition or combination formulation of the present invention can be administered systemically or locally and orally or parenterally when administered to a mammal (such as a human, a horse, a cow or a pig, preferably a human).

The pharmaceutical composition or combination formulation of the present invention can be prepared in a suitable form selected depending on the mode of administration, by a commonly used method for preparing various formulations.

In the present invention, the term "remission" and "remission state" are as defined in "the Guideline on Clinical Evaluation Method of Therapeutic Agent for Diabetic Nephropathy (draft)" presented by the Pharmaceutical and Food Safety Bureau of the Ministry of Health, Labour and Welfare as follows.

When a morning urine sample was measured twice consecutively, each of the measurement values corresponds to:

(i) a urine albumin value (urine albumin/Cr ratio) <30 mg/g Cr; and
(ii) a decrease by not less than 30% from the previous value.

"Diabetic nephropathy" is classified into disease stages of Stages 1 to 5 by the Japan Diabetes Society as follows.

Stage 1: pre-nephropathy stage
Stage 2: early nephropathy stage
Stage 3: overt nephropathy stage
Stage 4: renal failure stage
Stage 5: dialysis stage

In the present invention, "remission" is preferably a remission from Stage 2 to Stage 1.

Examples of the "excipient" to be used include a sugar derivative such as lactose, sucrose, glucose, mannitol or sorbitol; a starch derivative such as corn starch, potato starch, α-starch or dextrin; a cellulose derivative such as crystalline cellulose; gum arabic; dextran; or an organic excipient such as pullulan; or a inorganic excipient such as a silicate derivative such as light anhydrous silicic acid, synthetic aluminum silicate, calcium silicate or magnesium aluminometasilicate; a phosphate such as calcium hydrogen phosphate; a carbonate such as calcium carbonate; and a sulfate such as calcium sulfate.

Examples of the "lubricant" to be used include stearic acid; a metal stearate such as calcium stearate or magnesium stearate;; talc; colloidal silica; a wax such as beeswax or whale wax; boric acid; adipic acid; a sulfate such as sodium sulfate, glycol; fumaric acid; sodium stearyl fumarate; sodium benzoate; D, L-leucine; a lauryl sulfate such as sodium lauryl sulfate or magnesium lauryl sulfate; a silicic acid such as silicic anhydride or silicic acid hydrate; and the above-mentioned starch derivative.

Examples of the "binder" to be used include hydroxypropylcellulose, hydroxypropylmethylcellulose, polyvinylpyrrolidone and macrogol, and the same compounds as described for the excipient.

Examples of the "disintegrant" to be used include a cellulose derivative such as low substituted hydroxypropylcellulose, carboxymethylcellulose, carboxymethylcellulose calcium or internally cross-linked carboxymethylcellulose sodium; cross-linked polyvinylpyrrolidone; and a chemically modified starch or cellulose such as carboxymethyl starch or carboxymethyl starch sodium.

Examples of the "emulsifier" to be used include a colloidal clay such as bentonite or Veegum; a metal hydroxide such as magnesium hydroxide or aluminum hydroxide; an anionic surfactant such as sodium lauryl sulfate or calcium stearate; a cationic surfactant such as benzalkonium chloride; and a nonionic surfactant such as polyoxyethylene alkyl ether, polyoxyethylene sorbitan fatty acid ester or sucrose fatty acid ester.

Examples of the "stabilizer" to be used include a para-hydroxybenzoic acid ester such as methylparaben or propylparaben; an alcohol such as chlorobutanol, benzyl alcohol or phenylethyl alcohol; benzalkonium chloride; a phenol such as phenol or cresol; thimerosal; dehydroacetic acid; and sorbic acid.

Examples of the "flavoring agent" to be used include a sweetener such as saccharin sodium or aspartame; an acidulant such as citric acid, malic acid or tartaric acid; and a perfume such as menthol, lemon flavor or orange flavor.

Examples of the "diluent" to be used include those usually used as a diluent, such as lactose, mannitol, glucose, sucrose, calcium sulfate, calcium phosphate, hydroxypropylcellulose, microcrystalline cellulose, water, ethanol, polyethylene glycol, propylene glycol, glycerol, starch, polyvinyl pyrrolidone, magnesium aluminometasilicate or mixtures thereof.

The dose of (S)-1-(2-hydroxyethyl)-4-methyl-N-[4-(methylsulfonyl)phenyl]-5-[2-(trifluoromethyl)phenyl]-1H-pyrrole-3-carboxamide may vary according to various conditions such as the activity of the individual drug, the symptoms, age and body weight of the patient, and the like. The dose varies depending on the symptoms, age and the like, but in the case of oral administration, a lower limit per time of 0.1 mg (preferably 0.5 mg) and an upper limit per time of 20 mg (preferably 10 mg) and in the case of parenteral administration, a lower limit per time of 0.01 mg (preferably 0.05 mg) and an upper limit per time of 2 mg (preferably 1 mg) can be administered to an adult one to three times per day.

The dose of the angiotensin II receptor antagonist may vary according to various conditions such as the activity of the individual drug, the symptoms, age and body weight of the patient, and the like. The dose varies depending on the symptoms, age and the like, but in the case of oral administration, a lower limit per time of 0.1 mg (preferably 0.5 mg) and an upper limit per time of 100 mg (preferably 50 mg) and in the case of parenteral administration, a lower limit per time of 0.01 mg (preferably 0.05 mg) and an upper limit per time of 10 mg (preferably 5 mg) can be administered to an adult one to three times per day depending on the symptoms.

The dose of a calcium antagonist may vary according to various conditions such as the activity of the individual drug, the symptoms, age and body weight of the patient, and the like.
The dose varies depending on the symptoms, age of the patient and the like, but in the case of oral administration, a lower limit per time of 0.1 mg (preferably 0.5 mg) and an upper limit per time of 100 mg (preferably 50 mg) and in the case of parenteral administration, a lower limit per time of 0.01 mg (preferably 0.05 mg) and an upper limit per time of 10 mg (preferably 5 mg) can be administered to an adult one to three times per day depending on the symptoms.

When a plurality of drugs is administered, they can be administered simultaneously or separately at intervals depending on the symptoms.

## Examples

Hereinafter, the present invention will be further described in detail with reference to examples. However, these examples are not intended to limit the scope of the present invention.

<Example 1> Ameliorative effect on a renal disorder

Deoxycorticosterone acetate (DOCA)/sodium chloride-loaded hypertension model was prepared by the following procedure. 6 Week-old male WKY rats (WKY/Izm, SPF grade, produced by Funabashi Farm Co., Ltd.) were subjected to unilateral nephrectomy, diet containing 4% sodium chloride (4% NaCl-containing FR-2, manufactured by Funabashi Farm Co., Ltd.) was given from 7 weeks old by free feeding, and DOCA was administered subcutaneously once a week. DOCA was suspended in a 0.5% carboxymethylcellulose solution (20 mg/mL) and administered in an amount corresponding to 1 mL/kg. The group to which DOCA was not administered was designated as DOCA non-administration group and the group to which DOCA was administered was designated as DOCA administration group.

At 11 weeks old, some of the animals in the DOCA non-administration group were dissected and the other animals in the DOCA non-administration group were designated as normal group (Group 1). At the same time, some of the animals in the DOCA administration group were also dissected, and the others of the DOCA administration group were divided into the following groups (Groups 2 to 4) .

Group 1: normal group, n = 6 animals
Group 2; control group, n = 6 animals
Group 3; compound (Ia) administration group [compound (Ia) 3 mg/kg], n = 6 animals
Group 4: DOCA administration interruption + compound (Ia) administration group [compound (Ia) 3 mg/kg], n = 6 animals

The dosing schedule of test drugs for each group after 11 weeks is as follows.
To Group 1, oral administration of a 0.5% methylcellulose solution was continued for 4 weeks.
To Group 2, oral administration of 0.5% methylcellulose solution and subcutaneous administration (once a week) of DOCA were continued for 4 weeks.
To Group 3, oral administration of compound (Ia) suspended in 0.5% methylcellulose solution (2 mL/kg) and subcutaneous administration (once a week) of DOCA were continued for 4 weeks.
To Group 4, only oral administration of compound (Ia) suspended in 0.5% methylcellulose solution (2 mL/kg) was continued for 4 weeks.

At the end of 4 weeks of administration of the test drug or the like (at the age of 15 weeks old), all animals were dissected.

At 7, 11, 13 and 15 weeks old, 24-hour urine collections were conducted using metabolic cages (2100-R, Watanabe Isolator Systems Co., Ltd.). Daily urinary protein excretion was calculated from the urine protein concentration (measured by BiOLiS 24i premium [Tokyo Boeki Medisys Inc.]) and urine volume. The results are shown in Table 1.

[Table 1]

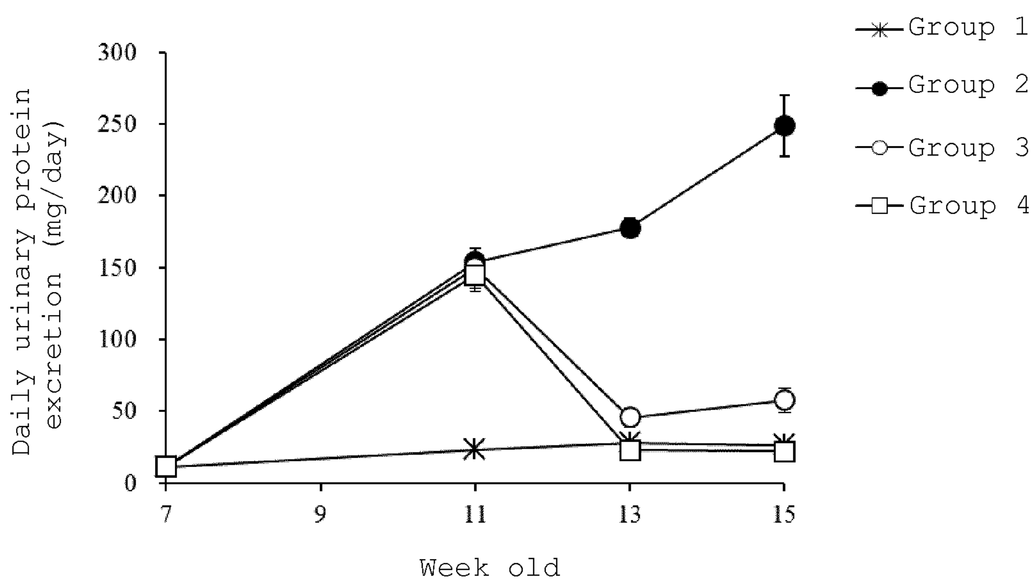

At the time of dissection at 11 and 15 weeks old, the kidneys were isolated and weighed, and the kidney weight per body weight was calculated. The results are shown in Table 2.

[Table 2]

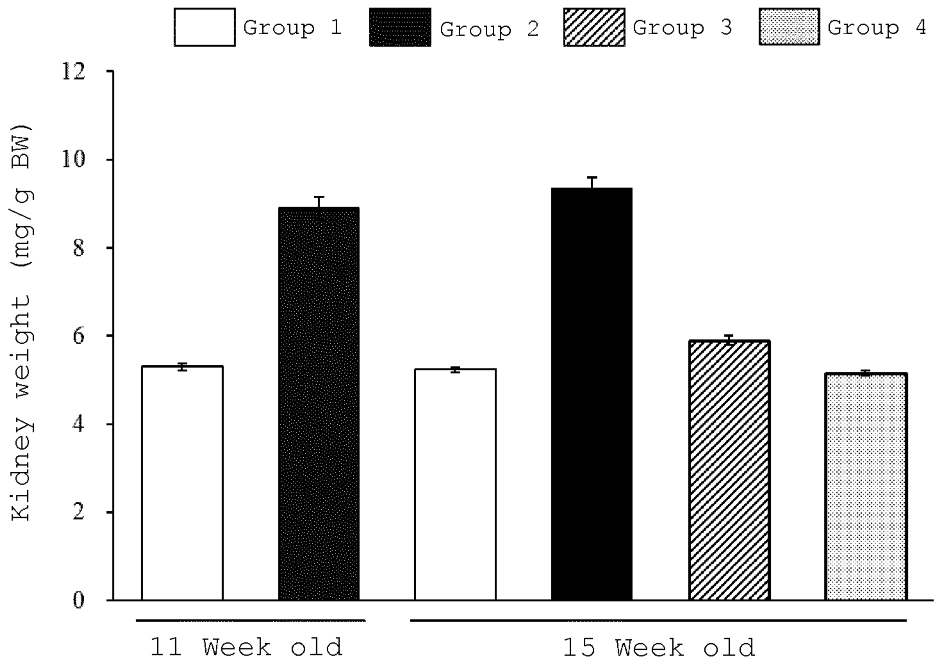


Furthermore, histopathological sections were prepared from a part of the isolated kidneys, and histopathological evaluation (scoring of glomerulosclerosis and tubular disorder) was conducted. For glomerulosclerosis, 30 glomeruli were selected at random for each sample, and the degree of glomerulosclerosis of each sample was quantified on the basis of 5 stages (0 to 4) to determine an average value of 30 values, which was used as a score of the sample (Uehara Y, et al., Hypertens. Res. 1992; 15, pp17-26). For tubular disorder, for each of the cortical and medullary sides, 10 fields of view were selected for each sample, the degree of tubular disorder was quantified on the basis of 5 stages (0 to 4) to determine the average value of values for each 10 fields of view, which was used as a score of the sample (Uehara Y, et al., Hypertens. Res. 1992; 15, pp17-26). Results are shown in Table 3 (each numerical value in the table represents the mean ± standard error).

[Table 3]

| Week old | Group | Glomerulosclerosis | Tubular disorder | |
|---|---|---|---|---|
| | | | Cortical side | Medullary side |
| 11 | Group 1 | 0.01 ± 0.01 | 0.43 ± 0.06 | 0.45 ± 0.05 |
| | Group 2 | 0.73 ± 0.10 | 1.62 ± 0.09 | 2.23 ± 0.05 |
| 15 | Group 1 | 0.06 ± 0.01 | 0.55 ± 0.06 | 0.58 ± 0.05 |
| | Group 2 | 1.59 ± 0.13 | 1.82 ± 0.09 | 2.57 ± 0.07 |
| | Group 3 | 0.29 ± 0.05 | 1.02 ± 0.07 | 1.73 ± 0.04 |
| | Group 4 | 0.20 ± 0.04 | 0.67 ± 0.06 | 1.42 ± 0.11 |

DOCA is known to act as a ligand for MR, and a DOCA/sodium chloride-loaded hypertension model is widely used as a disease model mediated by MR activation. The above results show that the disease model animals exhibited the excellent ameliorative effect for renal disorders (proteinuria, renal hypertrophy, glomerulosclerosis and tubular disorder) by administration of compound (Ia).

The results of Group 4 show that renal disorders were ameliorated almost to the normal group level by administration of compound (Ia) to the disease model animals in addition to further inhibition of MR activation by interruption

of DOCA administration (= interruption of MR activation). Since the MR activationinhibitory effect can be achieved to the level in the compound (Ia) administration with interruption of DOCA administration by adjusting the dosage of compound (Ia), the renal disorder can be ameliorated (remitted) to a state close to the normal group by only administration of compound (Ia).

Among renal diseases, diabetic nephropathy, glomerulonephritis (such as IgA nephropathy and mesangioproliferative glomerulonephritis) and nephrosclerosis are known as diseases involving MR activation in the onset and progress of the disease state. Therefore, for these diseases involving MR activation, it is understood that administration of compound (Ia) can ameliorate a renal disorder, which can reach a remission state even for renal diseases related to MR activation.

Furthermore, by administering a drug selected from an angiotensin II receptor antagonist and a calcium antagonist concurrently with or separately from compound (Ia), a combined effect such as an increase in remission rate is expected.

<Example 2> Clinical study of compound (Ia)

A clinical study targeted to patients with type 2 diabetes mellitus having albumin urine is conducted to evaluate the efficacy, safety or the like of compound (Ia) .
This study is a Phase II randomized, double-blind comparison study in which the placebo control group was randomly assigned. Each of the patients is subjected to a washout period of 4 to 12 weeks followed by treatment comprising administrating compound (Ia) (0.625 mg, 1.25 mg, 2.5 mg, 5 mg) once a day over a treatment period of 12 weeks.

Approximately 400 patients in approximately 70 facilities are expected to enter this study. The patient eligible for entry is as follows:

1. The patient is a patient with type 2 diabetes mellitus;
2. The patient is 20 years old or more at the time of consent;
3. Urine albumin/creatinine ratio (UACR) is 45 mg/g Cr or more and less than 300 mg/g Cr;
4. Estimated glomerular filtration rate from creatinine (eGFRcreat) is 30 mL/min/1.73 $m^2$ or more; and
5. The patient has been subjected to treatment with an angiotensin-converting enzyme inhibitor or an angiotensin II receptor antagonist (with an angiotensin-converting enzyme inhibitor or an angiotensin II receptor antagonist and optionally a calcium antagonist) since at least 3 months before the start of treatment.

The patient is followed up for approximately 6 weeks after the end of dosing, and evaluated for variation in UACR from baseline, remission rate of UACR and numerical value of eGFR as well as safety.

By conducting the above study, it is found that compound (Ia) can be administered to a patient, who does not obtain a sufficient therapeutic effect by treatment with an angiotensin-converting enzyme inhibitor or an angiotensin II receptor antagonist (with an angiotensin-converting enzyme inhibitor or an angiotensin II receptor antagonist and optionally a calcium antagonist), to achieve an excellent therapeutic effect (for example, a UACR reducing effect).

<Example 3> Clinical study of compound (Ia) (Phase II)

A clinical study targeted to patients with type 2 diabetes mellitus having albumin urine was conducted to evaluate the efficacy and safety of the like of compound (Ia). This study is a Phase II randomized, placebo controlled, double-blind comparison study. Each of the patients was subjected to a washout period of 4 to 12 weeks followed by treatment comprising administrating placebo or compound (Ia) (0.625 mg, 1.25 mg, 2.5 mg, 5 mg) once a day over a treatment period of 12 weeks.

365 Patients in Japan who met the following criteria entered this study, and an efficacy and safety analysis set included 358 cases.

1. The patient is with type 2 diabetes mellitus;
2. The patient is 20 years old or more at the time of consent;
3. Urine albumin/creatinine ratio (UACR) is 45 mg/g Cr or more and less than 300 mg/g Cr;
4. Estimated glomerular filtration rate calculated with serum creatinine (eGFRcreat) is 30 mL/min/1.73 $m^2$ or more; and
5. The patient has been subjected to treatment with an angiotensin-converting enzyme inhibitor or an angiotensin II receptor antagonist (and optionally a calcium antagonist) since at least 3 months before the start of treatment.

The patient was followed up for approximately 6 weeks after the end of dosing, and evaluated for change in UACR from baseline, remission rate of UACR and numerical value of eGFR as well as safety.

In the following, Table 4 shows the results of the reduction rate (geometric mean) of UACR from baseline at

the end of dosing, and Table 5 shows the result of the remission rate of UACR*1.

[Table 4]

| | Placebo | Compound (Ia) | | | |
|---|---|---|---|---|---|
| | | 0.625 mg | 1.25 mg | 2.5 mg | 5 mg |
| Reduction rate of UACR (%) | 7 | 21 | 37 | 50 | 56 |

[Table 5]

| | Placebo | Compound (Ia) | | | |
|---|---|---|---|---|---|
| | | 0.625 mg | 1.25 mg | 2.5 mg | 5 mg |
| Remission rate of UACR (%) | 3.0 | 7.5 | 12.3 | 21.2 | 21.1 |

*1) When UACR at both 11 weeks and 12 weeks was less than 30 mg/g Cr and decreased by 30% or more from UACR of the observation period, type 2 diabetes mellitus was considered to have been remitted, and the remission rate of UACR was calculated according to the following equation.

$$\text{Remission rate of UACR (\%)} = (\text{number of subjects in remission} \div \text{number of all subjects to be analyzed}) \times 100$$

From the above results, it has been found that compound (Ia) can be administered to a patient, who has not obtained a sufficient therapeutic effect by treatment with an angiotensin-converting enzyme inhibitor or an angiotensin II receptor antagonist (and optionally a calcium antagonist), to achieve an excellent therapeutic effect (for example, a UACR reducing effect and an increase in a remission rate).

Industrial Applicability

According the present invention, a medicine for treatment of a renal disease involving the mineralocorticoid receptor (particularly, diabetic nephropathy) can be obtained.

## Claims

1. A pharmaceutical composition comprising a mineralocorticoid receptor antagonist or a pharmaceutically acceptable salt thereof, or a hydrate thereof, for treating a renal disease by ameliorating a renal disorder involving activation of the mineralocorticoid receptor.

2. A pharmaceutical composition comprising a mineralocorticoid receptor antagonist or a pharmaceutically acceptable salt thereof, or a hydrate thereof, for treating a renal disease to put it into a remission state by ameliorating a renal disorder involving activation of the mineralocorticoid receptor.

3. The pharmaceutical composition according to claim 1 or 2, wherein the renal disease is diabetic nephropathy, glomerulonephritis or nephrosclerosis.

4. The pharmaceutical composition according to claim 1 or 2, wherein the renal disease is diabetic nephropathy.

5. The pharmaceutical composition according to any one of claims 1 to 4, wherein the mineralocorticoid receptor antagonist is (S)-1-(2-hydroxyethyl)-4-methyl-N-[4-(methylsulfonyl)phenyl]-5-[2-(trifluoromethyl)phenyl]-1H-pyrrole-3-carboxamide.

6. The pharmaceutical composition according to any one of claims 1 to 5, further comprising one or more components

selected from an angiotensin II receptor antagonist and a calcium antagonist.

**7.** The pharmaceutical composition according to claim 6, wherein the one or more components selected from an angiotensin II receptor antagonist and a calcium antagonist is olmesartan medoxomil.

**8.** The pharmaceutical composition according to claim 6, wherein the one or more components selected from an angiotensin II receptor antagonist and a calcium antagonist is amlodipine.

**9.** A method for treating a renal disease to put it into a remission state by ameliorating a renal disorder involving activation of the mineralocorticoid receptor, comprising administering a mineralocorticoid receptor antagonist to a mammal.

**10.** The method according to claim 9, comprising administering to the mammal one or more components selected from an angiotensin II receptor antagonist and a calcium antagonist, concurrently with or separately from the mineralocorticoid receptor antagonist.

## INTERNATIONAL SEARCH REPORT

International application No. PCT/JP2017/011340

**A. CLASSIFICATION OF SUBJECT MATTER**

*A61K45/00*(2006.01)i, *A61K31/40*(2006.01)i, *A61K31/4178*(2006.01)i, *A61K45/06*(2006.01)i, *A61P3/10*(2006.01)i, *A61P5/42*(2006.01)i, *A61P13/12*(2006.01)i, *A61P43/00*(2006.01)i

According to International Patent Classification (IPC) or to both national classification and IPC

**B. FIELDS SEARCHED**

Minimum documentation searched (classification system followed by classification symbols)
A61K45/00, A61K31/40, A61K31/4178, A61K45/06, A61P3/10, A61P5/42, A61P13/12, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN), Clinical Trials Information(JAPIC), ClinicalTrials.gov(NIH)

**C. DOCUMENTS CONSIDERED TO BE RELEVANT**

| Category* | Citation of document, with indication, where appropriate, of the relevant passages | Relevant to claim No. |
|---|---|---|
| X<br>Y | ARAI K. et al., Pharmacological profile of CS-3150, a novel, highly potent and selective non-steroidal mineralocorticoid receptor antagonist, European Journal of Pharmacology, 2015, Vol.761, p.226-234 | 1-5,9<br>6-8 |
| X<br>Y | KOLKHOF P. et al., Nonsteroidal antagonists of the mineralocorticoid receptor, Curr Opin Nephrol Hypertens, 2015, Vol.24, p.417-424 | 1-5,9<br>6-8 |
| X<br>Y | YANG J. et al., Mineralocorticoid receptor antagonists - pharmacodynamics and pharmacokinetic differences, Current Opinion in Pharmacology, 2016 Mar 2nd, Vol.27, p.78-85 | 1-5,9<br>6-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 April 2017 (14.04.17) | 09 May 2017 (09.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/011340

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2006/123765 A1 (Sankyo Co., Ltd.), 23 November 2006 (23.11.2006), paragraph [0018] & JP 5000491 B2 & CN 101171006 A & KR 10-2008-0011394 A & KR 10-1318032 B & TW 200719891 A | 6,7 |
| Y | WO 2009/057569 A1 (Daiichi Sankyo Co., Ltd.), 07 May 2009 (07.05.2009), paragraph [0019] & CN 101842096 A & KR 10-2010-0072317 A & TW 200927197 A | 6,7 |
| Y | WO 2010/018777 A1 (Daiichi Sankyo Co., Ltd.), 18 February 2010 (18.02.2010), paragraph [0011] & JP 2015-17136 A & JP 5688799 B2 & TW 201010699 A | 6,7 |
| Y | JP 2003-528927 A (Pfizer Inc.), 30 September 2003 (30.09.2003), claim 3 & WO 2001/074390 A2 claim 3 & US 2001/0036954 A1 & US 2003/0109557 A1 & EP 1284719 A2 & AU 3951001 A & BR 109783 A & CA 2403950 A & PA 8514601 A & AR 27763 A & MX 02009819 A & PE 11632001 A | 6,8 |
| Y | JP 2004-516266 A (Novartis AG.), 03 June 2004 (03.06.2004), claim 1 & WO 2002/049645 A1 claim 1 & EP 1345607 A1 & DE 60122928 T & AU 2581601 A & AU 3262002 A & CA 2432282 A & NO 20032765 A & CZ 20031677 A & HU 302455 A & SK 7642003 A & BR 116228 A & PL 360989 A & NZ 526385 A & HK 1059566 A & CN 1461218 A & KR 10-2003-0061840 A & RU 2003121019 A & ZA 200304514 A & MX 03005462 A & SI 1345607 T & AT 319448 T & IL 156136 D & AU 2001225816 B & AT 338549 T & DK 1345607 T & ES 2272565 T | 6,8 |

Form PCT/ISA/210 (continuation of second sheet) (January 2015)

**INTERNATIONAL SEARCH REPORT**

International application No.
PCT/JP2017/011340

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2012-522006 A (Gupta Ajay), 20 September 2012 (20.09.2012), paragraphs [0116], [0142] & WO 2010/111599 A2 page 35, lines 13 to 17; page 39, lines 4 to 14 & US 2010/0249081 A1 & US 2015/0258062 A1 & EP 2411006 A2 & CA 2756779 A & AU 2010229771 A & CN 102421433 A | 6,8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006012642 A
- US 20080234270 A
- WO 2008056907 A
- US 20100093826 A
- JP 2082519 A
- US 5616599 A
- JP 1401088 A
- US 4572909 A
- JP H0578328 B
- JP S6323868 B
- US 5138069 A
- JP H04364171 B
- EP 459136 A
- US 5354766 A
- JP H04159718 B
- EP 433983 A
- JP H04506222 W
- WO 9114679 A
- US 5185351 A
- US 5591762 A
- JP H05271228 B
- US 5243054 A


### Non-patent literature cited in the description


- *Nephron. Experimental Nephrology,* 2014, vol. 126, 16-24
- *Journal of Nephrology,* 2013, vol. 26, 199-206
- *Journal of Translational Medicine,* 2011, vol. 9, 169
- *American Journal of Nephrology,* 2004, vol. 24, 242-9
- **UEHARA Y et al.** *Hypertens. Res.,* 1992, vol. 15, 17-26